# EUROPÄISCHE PATENTSCHRIFT

(11) **EP 2 473 204 B1**
(45) Veröffentlichungstag und Bekanntmachung des Hinweises auf die Patenterteilung: **28.11.2018**
(21) Anmeldenummer: 10762590.7
(22) Anmeldetag: 02.09.2010
(51) Int. Cl.: A61L 24/02, A61L 27/12, C01B 25/32

(54) **HYDROXYLAPATITMATERIAL SOWIE VERFAHREN ZU DESSEN HERSTELLUNG**
HYDROXYLAPATITE MATERIAL, AND METHOD FOR THE PRODUCTION THEREOF
MATÉRIAU D'HYDROXYLAPATITE ET SON PROCÉDÉ DE PRÉPARATION

(30) Priorität: 02.09.2009 DE 102009039665
(43) Veröffentlichungstag der Anmeldung: 11.07.2012
(73) Patentinhaber: OSARTIS GmbH, 65807 Dieburg (DE)
(72) Erfinder: DINGELDEIN, Elvira, 63933 Mönchberg (DE); HEIMANN, Lydia, 63768 Hösbach (DE); GASQUERES, Georgiana, 63739 Aschaffenburg (DE); WOLFSTÄDTER, Marco, 63939 Wörth/Main (DE)
(74) Vertreter: Herden, Andreas F.
(86) Internationale Anmeldenummer: PCT/EP2010/005383
(87) Internationale Veröffentlichungsnummer: WO 2011/026618

(56) Entgegenhaltungen:
- DE-A1- 3 531 144
- US-A- 6 013 591
- US-B1- 6 506 217

## Beschreibung

### Gebiet der Erfindung

Die Erfindung betrifft ein Hydroxylapatitmaterial sowie ein Verfahren zu dessen Herstellung.

### Hintergrund der Erfindung

Hydroxylapatit ist als Knochenersatzmaterial bekannt.

Insbesondere ist nanoskaliges Hydroxylapatit, also Hydroxalapatit, dessen mittlere Partikelgröße weniger als 100 nm beträgt, bekannt und welches als Paste zur Behandlung von Knochendefekten verwendet wird. Insbesondere wird Hydroxylapatit verwendet, welches in einer Längsrichtung der Partikel gemessen eine Länge von weniger als 200 nm aufweist. Die Partikel sind nadel- bzw. stäbchenförmig ausgebildet und haben eine geringe Breite, vorzugsweise weniger als 50 nm.

Weiter bekannt sind gepresste oder gesinterte Formkörper aus Hydroxylapatit, welche als Implantat eingesetzt werden können.

Bekannte Hydroxylapatitpasten haben den Nachteil einer recht geringen Formstabilität, die dazu führt, dass die Paste in vielen Fällen nur bedingt geeignet ist, da diese nach dem Einbringen aufgrund mechanischer Beanspruchungen wieder aus der Defektstelle herausgedrückt werden kann. Weiter ist die Handhabbarkeit derartiger Pasten in vielen Fällen schwierig.

Gesinterte Körper aus Hydroxylapatit besitzen dagegen keine Flexibilität und werden in der Regel nur langsam durch natürliches Knochenmaterial ersetzt.

Das Dokument, US 6,013,591 A zeigt die Herstellung eines nanokristallinen Hydroxylapatitmaterials, bei dem ein Hydroxylapatitpulver durch isostatisches Pressen geformt wird und bei Normaldruck für 2 Stunden auf 1000, 1100, 1200 oder 1300°C erhitzt wird.

### Aufgabe der Erfindung

Der Erfindung liegt demgegenüber die Aufgabe zugrunde, ein Hydroxylapatitmaterial bereit zu stellen, welches gut handhabbar ist und mit welchem sich insbesondere Defektstellen im Kieferkammbereich gut behandeln lassen. Eine weitere Aufgabe der Erfindung ist, die Löslichkeit gegenüber bekannten gesinterten Hydroxylapatitkörpern zu verbessern.

Das Hydroxylapatitmaterial soll eine gute Degradation aufweisen und des Weiteren gute Benetzungs- und Kohäsionseigenschaften aufweisen.

### Zusammenfassung der Erfindung

Die Erfindung wird bereits durch ein Verfahren zur Herstellung eines Hydroxylapatitmaterials nach Anspruch 1 sowie durch ein formstabiles Hydroxylapatitmaterial nach Anspruch 10 gelöst.

Bevorzugte Ausführungsformen und Weiterbildungen der Erfindung sind den jeweiligen Unteransprüchen zu entnehmen.

Die Erfindung betrifft zum Einen ein Verfahren zur Herstellung eines Hydroxylapatitgranulats.

Als Ausgangsmaterial wird nanoskaliges Hydroxylapatit verwendet und in Form einer Paste verwendet. Unter nanoskaligem Hydroxylapatit wird insbesondere Hydroxylapatit mit einer Partikelgröße von unter 100 nm verstanden oder weniger als 200 nm in Längsrichtung bei Verwendung von stäbchen- bzw. nadelförmigen Partikeln.

Vorzugsweise handelt es sich dabei um nadelförmige Hydroxylapatitpartikel. Derartige Partikel sowie deren Herstellung sind beispielsweise in der europäischen Patentschrift EP 1023035 B1, in der europäischen Patentschrift EP 1317261 B1 und in der europäischen Patentschrift EP 0664133 B1 beschrieben. Auf den Offenbarungsgehalt dieser Dokumente wird vollumfänglich Bezug genommen. Es ist insbesondere vorgesehen, als Ausgangsmaterial ein nanoskaliges Hydroxylapatit zu verwenden, wie es in einem der Dokumente beschrieben wird oder nach dessen Lehre hergestellt wird.

Es versteht sich, dass im Sinne der Erfindung unter nanoskaligem Hydoxylapatit die Partikelgröße von Einzelpartikeln verstanden wird, wie sie sich in der Transmissionselektronenmikroskopie darstellt. Es sind keine Agglomerate gemeint, die sich insbesondere in hochkonzentrierten Hydroxylapatitsuspensionen aufgrund von Agglomeration von Einzelpartikeln bilden können.

Gemäß der Erfindung wird das nanoskalige Hydroxylapatit bei einer Temperatur zwischen 400 und 1300 °C, vorzugsweise zwischen 500 und 1100 °C und besonders bevorzugt zwischen 600 und 1000°C getempert, also temperaturbehandelt. Insbesondere liegt die Tempertemperatur zwischen 600 und 950 °C.

Es hat sich gezeigt, dass durch das Tempern des Hydroxylapatits mit einer Temperatur, welche unterhalb der normalen Sintertemperatur zur Bereitstellung von Hydroxylapatitformkörpern liegt, es zu einem geringen Kristallwachstum kommt, so dass ein formstabiles Hydroxylapatitmaterial entsteht.

Das Hydroxylapatitmaterial wird insbesondere als formstabiles Granulat bereit gestellt, welches sich mit einem geeigneten Applikationswerkzeug in die Defektstelle einbringen lässt. Gegenüber sprödem, gesinterten Material ist das mit dem erfindungsgemäßen Verfahren hergestellte Granulat weicher und hat wesentlich bessere Kohäsionseigenschaften. Es lassen sich somit mit einem geeigneten Werkzeug Defektstellen auf besonders einfache Weise auffüllen.

Über Temperatur und Dauer des Tempervorgangs können je nach Einsatzzweck die gewünschten Eigenschaften eingestellt werden. So führt eine hohe Temperatur und eine lange Haltezeit in der Regel zu einer größeren Kristallgröße. Die Erfinder können zeigen, dass sich die typischerweise nadelförmigen Kristalle des nanoskaligen Hydroxylapatits zu größeren Kristallen verbinden. Dabei entstehen insbesondere interkonnektierende hantel- und/oder schuppenförmige Strukturen.

Vorzugsweise wird zum Tempern eine Hydroxylapatitpaste, also eine hochkonzentrierte Hydroxylapatitsuspension verwendet.

Insbesondere wird eine Paste mit einem Feststoffanteil von 10 bis 60, vorzugsweise 30 bis 50% verwendet.

Bei einer Ausführungsform der Erfindung wird die Paste vor dem Tempern zu einem Granulat geformt. Aus der Paste werden also Granulatgrünlinge hergestellt, welche in einem anschließenden Temperschritt verfestigt werden.

Der Wasseranteil der Paste kann derart eingestellt werden, dass sich die Paste einerseits mit einem- Granulator gut verarbeiten lässt und andererseits so zäh ist, dass die hergestellten Granulatkörner nicht zerlaufen.

Bei einer alternativen Ausführungsform des Verfahrens werden Blöcke, insbesondere Platten aus einer Hydroxylapatitpaste, getempert, welche sodann zu einem Granulat zerbrochen, insbesondere zermahlen, werden.

Weiter betrifft die Erfindung ein Verfahren zur Herstellung eines Hydroxylapatitmaterials, bei welchem die Hydroxylapatitpaste zu einem Grünling geformt wird, der die Form eines Implantats vorgibt. Bei dieser Ausführungsform der Erfindung wird also mittels einer geeigneten Form ein Formkörper aus einer Paste bereit gestellt, welcher getempert wird. Es versteht sich, dass das Tempern mit einer Reduktion des Volumens einhergehen kann.

Die Haltezeit liegt bei einer bevorzugten Ausführungsform der Erfindung zwischen 30 Minuten und 10 Stunden, bevorzugt zwischen 2 und 4 Stunden.

Die Erfinder haben herausgefunden, dass das Tempern bei Atmosphärendruck, insbesondere unter Luftatmosphäre, erfolgen kann. Es ist somit nicht notwendig, aber möglich, ein Vakuum anzulegen oder ein Schutzgas zu verwenden.

Vorzugsweise wird nanoskaliges Hydroxylapatit mit einer mittleren Partikelgröße von unter 200 nm, insbesondere von ca. 100 nm gemessen in einer Längsrichtung verwendet. Dabei haben die Hydroxylapatitkristalle vorzugsweise eine im Wesentlichen nadelförmige Gestalt, sind also in einer Raumrichtung länger als in den beiden anderen Raumrichtungen.

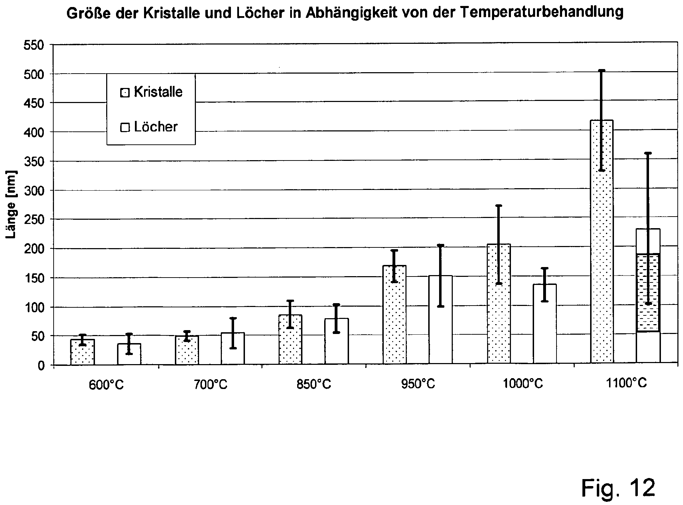

Mit der Erfindung lässt sich ein formstabiles Hydroxalapatitmaterial als Granulat bereitstellen, welches Kristalle mit einem mittleren Durchmesser von 20 bis 600 nm, vorzugsweise 40 bis 200 nm umfasst (siehe Fig. 12).

Die Größe des Granulats kann variabel eingestellt werden, insbesondere kann Granulat mit einem mittleren Durchmesser von 0,1 bis 10 mm, vorzugsweise 0,5 bis 5 mm bereitgestellt werden.

Das Hydroxylapatitgranulat mit einem mittleren Durchmesser zwischen 0,5 und 10 mm eignet sich insbesondere zur Behandlung von Knochendefekten, insbesondere im Kieferkammoder in spongiösen Knochendefekten z. B. des Femurs oder der Tibia.

Das Hydroxylapatitmaterial hat gemäß der Erfindung eine spezifische Oberfläche zwischen 3 und 40 m²/g.

Die spezifische Oberfläche kann über N₂-Adsorption gemessen werden, wobei die BET Isotherme verwendet wird, beispielsweise nach DIN ISO 9277.

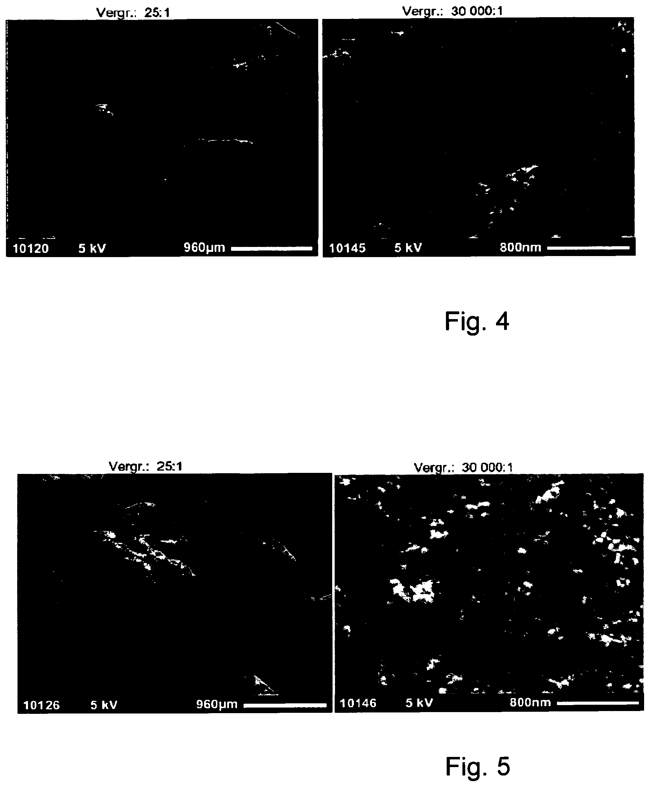

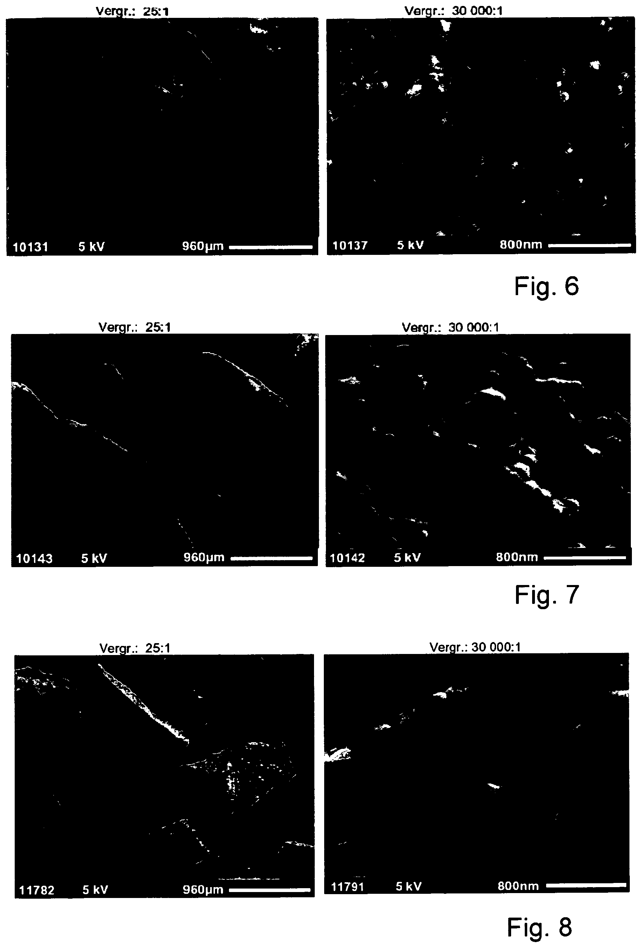

Die Erfindung ermöglicht also die Bereitstellung eines recht formstabilen Hydroxylapatitmaterials, welches eine relativ große Oberfläche aufweist und daher die Bildung von natürlichem Knochenmaterial besonders begünstigt. Das Verhältnis von spezifischer Oberfläche und Festigkeit kann über die Temperatur des Tempervorgangs eingestellt werden. So führen relativ niedrige Temperaturen von 600 °C zu einer spezifischen Oberfläche von über 35 m²/g, wogegen eine relativ hohe Temperatur von 1100 °C eine spezifische Oberfläche von weniger als 1 m²/g nach sich zieht. Die Reduzierung der spezifischen Oberfläche wird durch die Schrumpfung des Ausgangsmaterials während der thermischen Behandlung bewirkt. Dies führt zu einer Vergrößerung des Porendurchmessers und einer Verringerung der Zahl der Poren pro Fläche (Fig. 4, Fig. 7).

Bei dem erfindungsgemäßen Material handelt es sich vorzugsweise um nahezu reines Hydroxylapatitmaterial mit zumindest 90, vorzugsweise zumindest 95 % Hydroxylapatit (Gewichts%). Das Atomverhältnis von Calcium und Phosphor liegt vorzugsweise zwischen 1,6 und 1,7.

Das formstabile Hydroxylapatitmaterial kann in einen Applikator gefüllt werden.

Insbesondere handelt es sich dabei um einen Applikator mit einem Kolben, mittels dessen das Hydroxylapatitmaterial ausgedrückt werden kann.

### Beschreibung der Zeichnungen

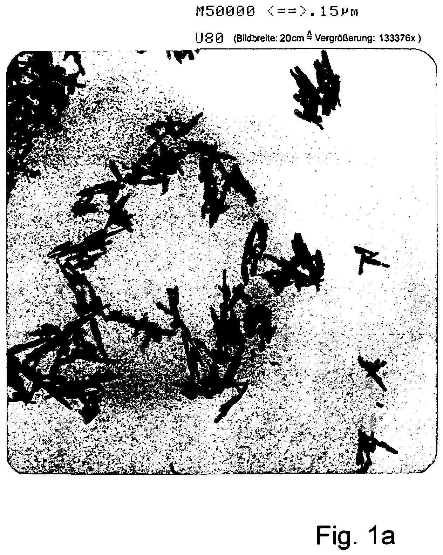

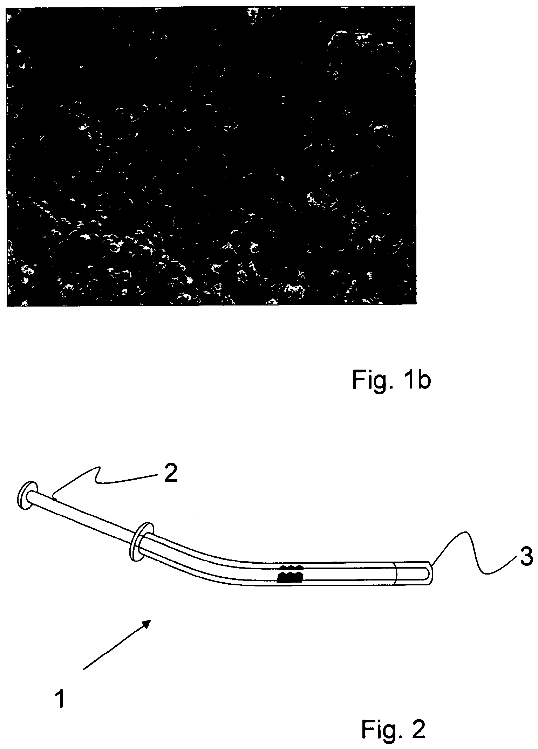

Die Erfindung soll im Folgenden Bezug nehmen auf die Zeichnungen Fig. 1 bis Fig. 12 näher erläutert werden.

Fig. 1a zeigt eine mikroskopische Ansicht des Ausgangsmaterials (nanoskaliger Hydroxylapatit). Das Hydroxylapatit liegt als Suspension mit nadelförmigen Partikeln vor. Die Länge (Längsrichtung) der einzelnen Partikel beträgt im Mittel weniger als 200 nm, vorzugsweise weniger als 100 nm.

Fig. 1b zeigt eine mikroskopische Ansicht eines Ausführungsbeispiels eines erfindungsgemäßen Hydroxylapatitgranulats. Zu erkennen sind interkonnektierende hantel- beziehungsweise schuppenförmige Partikel, die nadelförmige Struktur des Ausgangsmaterials liegt somit nicht mehr vor.

Das Material ist porös, leicht benetzbar und geht eine gute Kohäsion ein.

Infolge relativ kleiner Kristalle, welche ähnlich dem des natürlichen Knochenminerals sind, ist die Degradation des Materials verbessert.

Fig. 2 zeigt schematisch einen Applikator 1. Der Applikator weist einen Kolben 2 auf, welcher in einen Zylinder eingeführt wird, über den am Auslass 3 das Hydroxylapatitmaterial ausgedrückt werden kann.

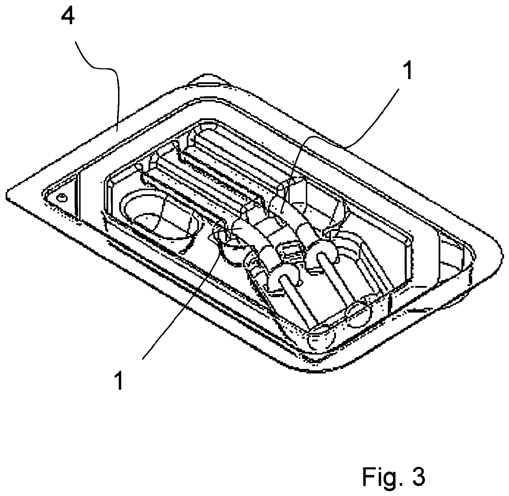

Die Applikatoren 1 können, wie in Fig. 3 dargestellt, vorgepackt in einer Schale 4 bereitgestellt werden.

Fig. 4 bis Fig. 8 zeigen REM Aufnahmen, bei welchen die Proben eines erfindungsgemäßen Hydroxylapatitmaterials mit einer Partikelgröße zwischen 1 und 2 mm mit Platin beschichtet und sodann untersucht wurden.

Gegenüber gestellt sind jeweils zwei Aufnahmen in einer 25-fachen (Bild jeweils links) und einer 30.000-fachen (Bild jeweils rechts) Vergrößerung.

Fig. 4 zeigt ein bei 600 °C getempertes Hydroxylapatitmaterial, Fig. 5 ein bei 700°C getempertes Hydroxylapatitgranulat, Fig. 6 ein bei 850 °C getempertes Hydroxylapatitgranulat, Fig. 7 ein bei 950 °C getempertes Hydroxylapatitmaterial und Fig. 8 ein bei 1100 °C getempertes Hydroxylapatitmaterial.

In der jeweils 25-fachen Vergrößerung ist zu erkennen, dass die Form des Granulats ähnlich ist, die Partikelgrößen der Granulate verringern sich mit steigender Temperatur.

In der 30.000-fachen Vergrößerung ist zu erkennen, dass die Kristalle mehr und mehr verschmelzen und sich dadurch vergößern. Auch die Kristallitgröße (bestimmt mittels XRD) nimmt mit der Temperatur zu: sie beträgt bei 600 °C etwa 20 bis 30 nm und erreicht 40 bis 50 nm bei 950 °C. Die Erfinder haben festgestellt, dass die Kristallitgröße hierbei nicht nur durch die Temperatur beeinflusst wird, sondern auch durch die Größe der Grünlinge vor der thermischen Behandlung. Das bei 950 °C getemperte Material hat eine kleinere Oberfläche und eine höhere Festigkeit. Bei einer Tempertemperatur von 1100 °C ist die nadelförmige Form der einzelnen Kristallite durch das Versintern weitgehend verloren gegangen.

Die Festigkeit von Granulat, getempert bei 600°C und 950 °C wurde mittels Friabilitätstest gemäß der Europäischen Pharmakopoeia 6 (Kapitel 2.9.41, Methode B) verglichen. Dabei zeigte sich für das Granulat, welches mit 600 °C getempert wurde, eine Friabilität F von etwa 260, ein bei 950 °C getempertes Material hat eine Friabilität F von etwa 155.

Die Friabilität kann als Maß für die Verarbeitbarkeit des Granulates je nach gewünschter Indikation dienen.

Gemäß der Erfindung liegt die Friabilität F zwischen 50 und 500.

Abhängig von der Indikation kann unter anderem über die Wahl der Tempertemperatur zumindest die Festigkeit eingestellt werden.

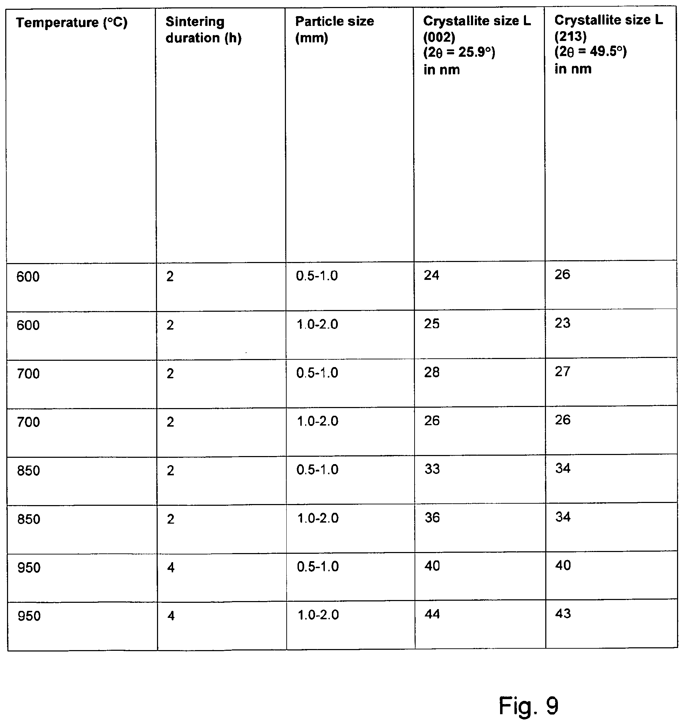

Fig. 9. zeigt eine Tabelle, in welcher die Kristallitgröße verschiedener Proben wiedergegeben ist. Die Proben sind dabei nach Temperatur, Temperzeit (sintering duration)und Partikelgröße geordnet. Die Kristallitgröße kann beispielsweise über Röntgenstrahlendiffraktion und Vergleich mit der JCPDS-Datenbank für Hydroxylapatit bestimmt werden. Die Kristallit- bzw. Korngröße steigt mit steigender Temperatur.

Die Kristallitgröße kann mittels der Scherrer-Formel berechnet werden, wobei für die Erstellung der in Fig. 9 wiedergegebenen Tabelle zwei Peaks für die Berechnung verwendet wurden: 2θ = 25.9° (Miller index(002)) und 2θ = 49.5° (Miller index (213)).

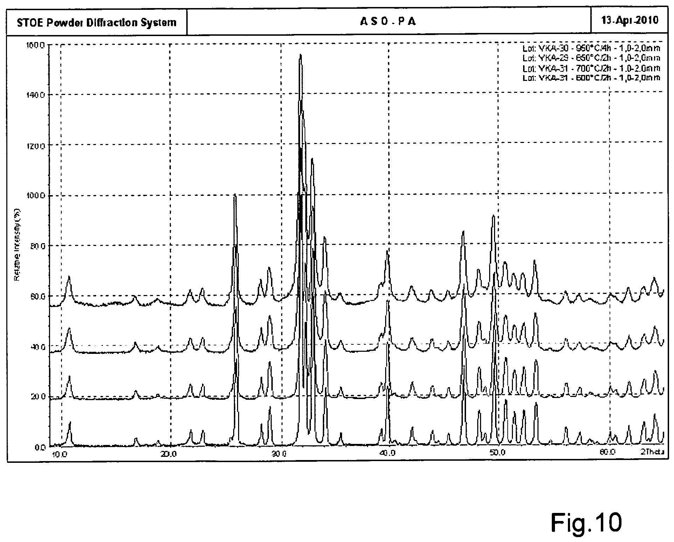

Fig. 10 zeigt eine derartige Diffraktionsmessung mit einem Diffraktometer der Handelsbezeichnung Stadi-P des Unternehmens Stoe & CIE. Wiedergegeben ist ein Winkel zwischen 9° und 65° (2*Theta).

Die X-Achse gibt den Winkel (2 Theta) und die Y-Achse die relative Intensität wieder. Gegenübergestellt sind 4 Kurven eines mit unterschiedlicher Temperatur gesinterten Hydroxylapatitmaterials, wobei von der obersten ausgehend bis zur untersten Kurve das Material bei folgenden Temperaturen getempert wurde: 600°C, 700°C, 850°C, 950°C.

Zu erkennen ist, dass der Kristallisierungsgrad mit steigender Tempertemperatur zunimmt, was sich in einer Verringerung der Peakbreite äußert.

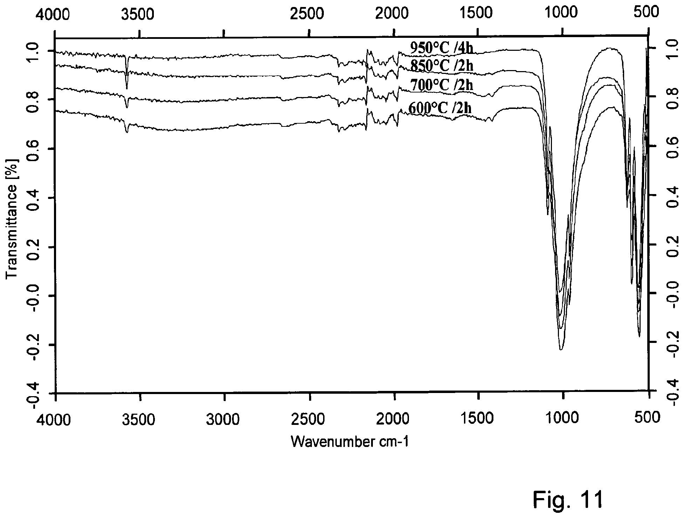

Fig. 11 zeigt schematisch einen FT-Infrarotspektroskopieverlauf verschiedener Granulatmaterialien, die ebenfalls bei Temperaturen zwischen 600°C (unterste Kurve) und 950°C (oberste Kurve) getempert wurden. Es wurden ein ATR-Gerät der Firma Specac Graseby und ein IFS28-Spektrometer der Firma Bruker verwendet.

Die infrarotspektroskopischen Aufnahmen konnten zeigen, dass nahezu reines Hydroxylapatitmaterial vorliegt. Bei Temperaturen zwischen 600°C und 850°C gibt es allenfalls einen kleinen Peak aufgrund eingeschlossener Kohlenstoffverbindungen. Durch höhere Temperaturen werden die Kohlenstoffverbindungen vollständig entfernt.

Fig. 12 zeigt die Größe der Hydroxylapatitkristalle in Abhängigkeit von der Temperatur während des Temperns. Auf der x-Achse sind bei unterschiedlichen Temperaturen getemperte Granulate aufgetragen und auf der y-Achse die Länge der Kristalle (jeweils links) und Löcher bzw. Poren (jeweils rechts) gegenüber gestellt. Zu erkennen ist, dass sich durch eine zunehmende Versinterung sowohl die Länge der Kristalle als auch der Durchmesser der Löcher erhöht. So steigt sowohl die mittlere Kristallgröße -als auch der Lochdurchmesser von unter 50 nm bei einer Tempertemperatur von 600 °C auf über 200 nm bei einer Tempertemperatur von 1100 °C.

Durch die Erfindung konnte ein formstabiles, leicht zu verarbeitendes Hydroxylapatitgranulat bereit gestellt werden, welches das Einwachsen von natürlichem Knochenmaterial fördert und welches sich leicht verarbeiten lässt.

## Patentansprüche

1. Verfahren zur Herstellung eines Hydroxylapatitgranulats, wobei nanoskaliges Hydroxylapatit bei einer Temperatur zwischen 400 und 1300 °C, vorzugsweise zwischen 500 und 1100 °C, besonders bevorzugt zwischen 600 und 1000 °C getempert wird, wobei ein Hydroxylapatitgranulat mit einer Friabilität F zwischen 50 und 500 hergestellt wird.

2. Verfahren zur Herstellung eines Hydroxylapatitgranulats nach dem vorstehenden Anspruch, **dadurch gekennzeichnet, dass** eine Hydroxylapatitpaste verwendet wird.

3. Verfahren zur Herstellung eines Hydroxylapatitgranulats nach dem vorstehenden Anspruch, **dadurch gekennzeichnet, dass** die Paste einen Feststoffanteil von 10 bis 60, vorzugsweise 30 bis 50 % aufweist.

4. Verfahren zur Herstellung eines Hydroxylapatitgranulats nach einem der vorstehenden Ansprüche, **dadurch gekennzeichnet, dass** eine Hydroxylapatitpaste vor dem Tempern zu einem Granulat geformt wird.

5. Verfahren zur Herstellung eines Hydroxylapatitgranulats nach einem der Ansprüche 1 bis 3, **dadurch gekennzeichnet, dass** Grünlinge aus einer Hydroxylapatitpaste in Form von Blöcken oder Granulat getempert werden.

6. Verfahren zur Herstellung eines Hydroxylapatitgranulats nach einem der Ansprüche 1 bis 3, **dadurch gekennzeichnet, dass** eine Hydroxylapatitpaste zu einem Grünling geformt wird, der die Form eines Implantats vorgibt.

7. Verfahren zur Herstellung eines Hydroxylapatitgranulats nach dem vorstehenden Anspruch, **dadurch gekennzeichnet, dass** die Hydroxylapatitpaste in einer Form getempert wird.

8. Verfahren zur Herstellung eines Hydroxylapatitgranulats nach einem der vorstehenden Ansprüche, **dadurch gekennzeichnet, dass** die Haltezeit beim Tempern zwischen 30 min und 10 h, bevorzugt zwischen 2 und 4 h liegt und/oder dass das Tempern bei Atmosphärendruck erfolgt und/oder dass nanoskaliges Hydroxylapatit mit einer Partikelgröße von weniger als 200 nm, gemessen in Richtung einer Längsachse, verwendet wird.

9. Verfahren zur Herstellung eines Hydroxylapatitgranulats nach einem der vorstehenden Ansprüche, **dadurch gekennzeichnet, dass** nanoskaliges Hydroxylapatit mit im Wesentlichen nadelförmigen Kristallen verwendet wird.

10. Formstabiles Hydroxylapatitgranulat, umfassend Kristalle mit einem mittleren Durchmesser von 20 bis 600 nm, vorzugsweise 40 bis 200 nm, besonders bevorzugt 50 bis 100 nm, und welches eine spezifische Oberfläche von 3 bis 40 m²/g aufweist und wobei die Friabilität F des Hydroxylapatitgranulats zwischen 50 und 500 liegt.

11. Formstabiles Hydroxylapatitgranulat nach Anspruch 10, **dadurch gekennzeichnet, dass** das Granulat mit einem mittleren Durchmesser zwischen 0,1 und 10 mm, besonders bevorzugt zwischen 0,5 und 5 mm vorliegt, und/oder dass das Hydroxylapatitgranulat zumindest 90, vorzugsweise zumindest 95 % Hydroxylapatit umfasst, und/oder dass das Atomverhältnis von Calcium zu Phosphor zwischen 1,6 und 1,7 liegt.

12. Formstablies Hydroxylapatitgranulat nach einem der Ansprüche 10 oder 11, **dadurch gekennzeichnet, dass** die Friabilität F des Hydroxylapatitgranulats zwischen 80 und 300 liegt.

13. Formstabiles Hydroxylapatitgranulat nach einem der Ansprüche 10 bis 12, **dadurch gekennzeichnet, dass** das Hydroxylapatitgranulat mit einem Verfahren nach einem der Ansprüche 1 bis 9 hergestellt wurde

14. Applikator, gefüllt mit formstabilem Hydroxylapatitmaterial nach einem der Ansprüche 10 bis 13, insbesondere Applikator, der einen Kolben zum Ausdrücken des Hydroxylapatitmaterials umfasst.

## Claims

1. A method for producing a hydroxylapatite granulate, wherein nanoscale hydroxylapatite is subjected to a thermal treatment at a temperature between 400 °C and 1300 °C, preferably between 500 °C and 1100 °C, more preferably between 600 °C and 1000 °C, thereby producing a hydroxylapatite granulate with a friability F between 50 and 500.

2. The method for producing a hydroxylapatite granulate according to the preceding claim, **characterized in that** a hydroxylapatite paste is used.

3. The method for producing a hydroxylapatite granulate according to the preceding claim, **characterized in that** the paste has a solids content from 10 to 60 %, preferably from 30 to 50 %.

4. The method for producing a hydroxylapatite granulate according to any one of the preceding claims, **characterized in that** a hydroxylapatite paste is formed into a granulated material prior to said thermal treatment.

5. The method for producing a hydroxylapatite granulate according to any one of claims 1 to 3, **characterized in that** hydroxylapatite paste green bodies in form of blocks or granulate are subjected to said thermal treatment.

6. The method for producing a hydroxylapatite granulate according to any one of claims 1 to 3, **characterized in that** a hydroxylapatite paste is formed into a green body which defines the shape of an implant.

7. The method for producing a hydroxylapatite granulate according to the preceding claim, **characterized in that** said hydroxylapatite paste is subjected to the thermal treatment within a mold.

8. The method for producing a hydroxylapatite granulate according to any one of the preceding claims, **characterized in that** the holding time during the thermal treatment is from 30 min to 10 h, preferably from 2 to 4 h, and/or that the thermal treatment is performed at atmospheric pressure, and/or that nanoscale hydroxylapatite is used which has a particle size of less than 200 nm as measured along a longitudinal axis thereof.

9. The method for producing a hydroxylapatite granulate according to any one of the preceding claims, **characterized in that** nanoscale hydroxylapatite is used which comprises substantially needle-shaped crystals.

10. A form-stable hydroxylapatite granulate, comprising crystallites having an average diameter from 20 to 600 nm, preferably from 40 to 200 nm, more preferably from 50 to 100 nm, and having a specific surface area from 3 to 40 m²/g, and wherein the friability F of the hydroxylapatite granulate is between 50 and 500.

11. The form-stable hydroxylapatite granulate according to claim 10, **characterized in that** the granulate is provided with an average diameter between 0.1 and 10 mm, more preferably between 0.5 and 5 mm, and/or that the hydroxylapatite granulate comprises at least 90 %, preferably at least 95 % of hydroxylapatite, and/or that the atomic ratio of calcium to phosphorus is between 1.6 and 1.7.

12. The form-stable hydroxylapatite granulate according to any one of claims 10 or 11, **characterized in that** the friability F of the hydroxylapatite granulate is between 80 and 300.

13. The form-stable hydroxylapatite granulate according to any one of claims 10 to 12, **characterized in that** the hydroxylapatite granulate was produced by a method according to any one of claims 1 to 9.

14. An applicator filled with form-stable hydroxylapatite material according to any one of claims 10 to 13, in particular an applicator comprising a plunger for pressing out said hydroxylapatite material.

## Revendications

1. Procédé de production d'un granulé d'hydroxyapatite, dans lequel l'hydroxyapatite de taille nanométrique est recuite à une température comprise entre 400 et 1300°C, de préférence entre 500 et 1100°C, de manière particulièrement préférée entre 600 et 1000°C, dans lequel un granulé d'hydroxyapatite ayant une friabilité F comprise entre 50 et 500 est produit.

2. Procédé de production d'un granulé d'hydroxyapatite selon la revendication précédente, **caractérisé en ce qu'**une pâte d'hydroxyapatite est utilisée.

3. Procédé de production d'un granulé d'hydroxyapatite selon la revendication précédente, **caractérisé en ce que** la pâte présente une proportion de matières solides de 10 à 60, de préférence 30 à 50 %.

4. Procédé de production d'un granulé d'hydroxyapatite selon l'une des revendications précédentes, **caractérisé en ce qu'**une pâte d'hydroxyapatite est formée en un granulé avant le recuit.

5. Procédé de production d'un granulé d'hydroxyapatite selon l'une des revendications 1 à 3, **caractérisé en ce que** des compacts verts constitués d'une pâte d'hydroxyapatite sont recuits sous forme de blocs ou de granulés.

6. Procédé de production d'un granulé d'hydroxyapatite selon l'une des revendications 1 à 3, **caractérisé en ce qu'**une pâte d'hydroxyapatite est formée en un compact vert prédéfinissant la forme d'un implant.

7. Procédé de production d'un granulé d'hydroxyapatite selon la revendication précédente, **caractérisé en ce que** la pâte d'hydroxyapatite est recuite en une forme.

8. Procédé de production d'un granulé d'hydroxyapatite selon l'une des revendications précédentes, **caractérisé en ce que** le temps de maintien lors du recuit est compris entre 30 min et 10 h, de préférence entre 2 et 4 h, et/ou **en ce que** le recuit s'effectue sous pression atmosphérique et/ou **en ce que** de l'hydroxyapatite de taille nanométrique ayant une taille de particules inférieure à 200 nm, mesurée dans la direction d'un axe longitudinal, est utilisée.

9. Procédé de production d'un granulé d'hydroxyapatite selon l'une des revendications précédentes, **caractérisé en ce que** de l' hydroxyapatite comportant des cristaux sensiblement en forme d'aiguilles est utilisée.

10. Granulé d'hydroxyapatite de forme stable, comprenant des cristaux ayant un diamètre moyen de 20 à 600 nm, de préférence 40 à 200 nm, de manière particulièrement préférée de 50 à 100 nm, et qui présente une surface spécifique de 3 à 40 m²/g et où la friabilité F du granulé d'hydroxyapatite est comprise entre 50 et 500.

11. Granulé d'hydroxyapatite de forme stable selon la revendication 10, le granulé étant **caractérisé en ce qu'**il est présent à un diamètre moyen compris entre 0,1 et 10 mm, de manière particulièrement préférée entre 0,5 et 5 mm, et/ou le granulé d'hydroxyapatite comprend au moins 90, de préférence au moins 95 % d'hydroxyapatite, et/ou **en ce que** le rapport atomique du calcium sur le phosphore est compris entre 1,6 et 1,7.

12. Granulé d'hydroxyapatite de forme stable selon l'une des revendications 10 ou 11, **caractérisé en ce que** la friabilité F du granulé d'hydroxyapatite est comprise entre 80 et 300.

13. Granulé d'hydroxyapatite de forme stable selon l'une des revendications 10 à 12, le granulé d'hydroxyapatite étant **caractérisé en ce qu'**il a été produit avec un procédé selon l'une des revendications 1 à 9.

14. Applicateur, rempli de matière d'hydroxyapatite de forme stable selon l'une des revendications 10 à 13, en particulier applicateur qui comprend un piston pour exprimer la matière d'hydroxyapatite.
